(19)

**Europäisches Patentamt**
**European Patent Office**
**Office européen des brevets**

(11) **EP 3 995 133 A1**

(12) # EUROPEAN PATENT APPLICATION

(43) Date of publication:
**11.05.2022 Bulletin 2022/19**

(21) Application number: **20382966.8**

(22) Date of filing: **09.11.2020**

(51) International Patent Classification (IPC):
**A61K 9/107** (2006.01)   **A61K 31/26** (2006.01)
**A61K 47/14** (2017.01)   **A61K 47/26** (2006.01)

(52) Cooperative Patent Classification (CPC):
**A61P 35/00; A61K 9/1075; A61K 31/26;**
**A61K 47/14; A61K 47/26**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Fundación Tecnalia Research & Innovation**
**20009 Donostia-San Sebastian, Guipuzcoa (ES)**

(72) Inventors:
- **Díaz De Apodaca Díaz, Elena**
  **01510 MIÑANO (ES)**

- **Fernández De Castro, Laura**
  **01510 MIÑANO (ES)**
- **Hidalgo Lemus, Noelia**
  **01510 MIÑANO (ES)**
- **San Vicente Laurent, Leire**
  **01510 MIÑANO (ES)**
- **Goñi de Cerio, Felipe**
  **48170 ZAMUDIO (ES)**
- **Gómez Fernández, Paloma**
  **48170 ZAMUDIO (ES)**

(74) Representative: **ZBM Patents - Zea, Barlocci & Markvardsen**
**Rambla Catalunya, 123**
**08008 Barcelona (ES)**

(54) **STABLE NANOEMULSIONS**

(57)    The invention provides a liquid oil/in/water (O/W) nanoemulsion comprising a continuous aqueous phase and dispersed oil droplets, said droplets having an average size comprised from 30 to 100 nm, and a zeta potential absolute value equal or higher than 30 mV.

The invention also provides processes for its preparation and uses thereof.

EP 3 995 133 A1

**Description**

**Technical Field**

The present invention relates to the field of nanoemulsions. In particular, the present invention relates to stable oil-in-water nanoemulsion compositions which can efficiently encapsulate poorly water-soluble compounds, to processes for their preparation, as well as to uses thereof.

**Background Art**

Emulsions are colloidal systems which have application in many industrial products such as food, cosmetics and pharmaceuticals. Oil-in-water emulsions are made of oil droplets which are dispersed in an aqueous continuous phase. One of the uses of emulsions in industry is to deliver active ingredients and components, such as, flavours, colours, vitamins, antioxidants, anti-microbials, pesticides, herbicides, cosmetics, nutraceuticals, phytochemicals and pharmaceuticals.
The active components can be oil soluble or water soluble, although their solubility in these environments can vary from highly soluble to poorly soluble. Administering active components that are not soluble in water poses a challenge as it requires the use of an appropriate vehicle for bringing an effective amount of the active component into the desired place of action. Oil-in-water emulsions are commonly used for the delivery of active components that are not soluble in water. Active components that are soluble in oil are dissolved/dispersed within the oil phase of the emulsion.
The emulsions that are conventionally used to deliver active components suffer from a number of significant limitations and disadvantages. Emulsions are kinetically stable structures that are subject to destabilisation through a number of mechanisms, ultimately resulting in complete phase separation of the emulsion. The tendency of emulsions to physically alter over time presents problems for their storage and handling. Furthermore, this physical degradation increases the likelihood that the preparation is in a sub-optimal state when physically administered.
The size (diameter) of a conventional oil-in-water emulsion ranges from several hundred nanometers to several microns. Since these particles are in the order of or greater than the wavelength of light, they have an opaque appearance. This has the disadvantage of altering the optical clarity of any product that the emulsion is incorporated into, reducing visual appeal. Furthermore, emulsions of this size have a low interfacial area to volume ratio. This has a negative impact on the emulsions ability to dissolve poorly soluble bioactives which are soluble at an interface.
Therefore, in spite of the efforts made there is still the need of stable emulsions which efficiently encapsulates poorly water-soluble compounds.

**Summary of Invention**

The present inventors have developed an oil/in/water (O/W) nanoemulsion, wherein the oil droplets have an average size of less than 100 nm, maintains good stability, and eliminates the use of undesirable alcohol as co-solvent in the aqueous phase.
Thus, in a first aspect the present invention provides an oil/in/water (O/W) liquid nanoemulsion comprising a continuous aqueous phase and a fatty phase of dispersed oil droplets, said droplets:

(a) having an average size comprised from 30 to 100 nm, and a zeta potential absolute value equal or higher than 30 mV, particularly comprised from 30 to 70 mV, particularly from 30 to 60 mV, particularly from 35 to 50 mV, particularly from 35 to 45 mV; and

(b) optionally encapsulating one or more, particularly one or two, poorly water-soluble compound(s), wherein a compound is considered to be "poorly water-soluble" when an amount equal or lower than 1 g, particularly equal or lower than 0.1 g, is dissolved in 100 mL of water at 20°C.

As it is shown below, the oil/in/water nanoemulsion of the invention with average droplet size of equal or lower than 100 nm, unexpectedly gives extremely favourable particle size distribution, optical clarity, and product stability.
Without being bound to the theory, the present inventors believed that this is due to the reduced size of the oily droplets but also to the zeta potential value shown by the oil droplets forming the nanoemulsion.
On one hand, the small particle size favours the stability of the nanoemulsion against flocculation or coalescence phenomena.
On the other hand, the zeta potential value of equal or higher than 30 mV guarantees the presence of strong repulsive forces instead of attractive forces between the oil droplets, this drop-by-drop repulsion preventing the coagulation and/or coalescence of the dispersed phase of the emulsions. The zeta potential value, therefore, guarantees the physical stability of the nanoemulsion during storage.

This strong stability was confirmed by subjecting a freshly prepared nanoemulsion of the invention to a centrifugation process for 30 min and 1500 x g, at 5°C and 23°C. In both cases, it was observed that both the particle size and the polydispersity index did not vary significantly, indicating that the nanoemulsion was stable, thanks to such repulsion forces.

Poorly water-soluble, therapeutically active agents and others (such as flavours, colours, vitamins, antioxidants, anti-microbials, pesticides, herbicides, cosmetics, nutraceuticals, and phytochemicals) can be incorporated into the nanoemulsion of the invention to improve their solubility and stability in aqueous medium and for active component delivery. As it is shown below, the nanoemulsion of the invention allows a high loading of the poorly water-soluble compound, due to the high interfacial area to volume ratio which can aid in the dissolution of poorly soluble bioactives and aid the rapid digestion of the emulsion by faster rates of lipolysis.

Furthermore, unlike many microemulsions, nanoemulsions retain their structure (small size) upon dilution and/or acidification. This may have the added benefit of aiding active adsorption as it is currently thought that emulsions below 100 nm have a greater ability to penetrate epithelial layers such as the skin and oral mucosa.

Another advantage of the nanoemulsions of the invention is that their preparation requires the use of a significantly lower amount of surfactant compared to microemulsions. This gives the nanoemulsions the advantage that less surfactant is incorporated upon addition of a certain amount of active/oil. This is beneficial from a toxicological, regulatory and taste perspective.

In a second aspect the present invention provides a O/W liquid nanoemulsion comprising a continuous aqueous phase and fatty phase of dispersed oil droplets, wherein

- the fatty phase is at % by weight with respect the total weight of the nanoemulsion (w/w) comprised from 9 to 11, particularly from 9.5 to 10.5;

- the aqueous phase is at a % by weight with respect the total weight of the nanoemulsion (w/w) comprised from 75 to 85, particularly from 77 to 83%, particularly from 78 to 82%;

the nanoemulsion further comprising a surfactant system:

(a) which has a total hydrophilic-lipophilic balance (HLB) value from 11-15, particularly from 12-14,

(b) is at a % (w/w) with respect to the total weight of the nanoemulsion comprised from 8 to 10%, particularly from 8.5 to 10%, and

the oil droplets optionally encapsulating one or more poorly water-soluble compound(s) as defined above; provided that the sum of the components does not exceed the 100%.

In a third aspect, the present invention provides a process for preparing a nanoemulsion as defined in the first aspect of the invention, the process comprising the steps of (a) mixing the aqueous and fatty phases, the fatty phase optionally including the poorly water-soluble compound as defined above, and a surfactant system, at a speed of at least 5000 rpm at room temperature, followed by (b) sonicating or subjecting to ultrasounds the resulting coarse emulsion from step (a).

In a fourth aspect the present invention provides a nanoemulsion obtainable by the process of the third aspect of the invention.

In a fifth aspect the present invention provides a nutraceutical composition comprising the nanoemulsion of the first aspect of the invention.

In a sixth aspect the present invention provides a functional food product comprising the nanoemulsion of the first aspect of the invention.

As it is shown below, the nanoemulsion of the invention is not only capable of loading high amounts of the poorly water-soluble compound, such as a drug, but also appropriately retain it until its use and deliver it at the desired moment, to exert its effect.

Thus, in a seventh aspect the invention provides the nanoemulsion of the invention (as defined under the first, second or fourth aspect) as a delivery system of poorly water-soluble pharmaceutical compounds.

The examples provided below shown that a nanoemulsion of the invention, encapsulating sulforaphane as poorly water-soluble compound, which is a pharmaceutical compound, is effective in reducing the proliferation of cancer cells. This means that the nanoemulsion of the invention does not negatively affect the release of the compound because a significant therapeutic effect is found.

Thus, in an eighth aspect the present invention provides a nanoemulsion as defined in the first, second or fourth aspects of the invention, which encapsulates one or more poorly water-soluble compounds which are pharmaceutical,

for use in therapy.

In a ninth aspect, the present invention provides a nanoemulsion as defined in the first, second or fourth aspect of the invention, which encapsulates one or more poorly water-soluble compounds which are pharmaceutical, for use in the treatment or prevention of cancer. This aspect can also be formulated as the use of a nanoemulsion as defined in the first second or fourth aspect of the invention, which encapsulates one or more poorly water-soluble compounds which are pharmaceutical, for the manufacture of a medicament for the treatment or prevention of cancer. This aspect can also be formulated as a method for the treatment or prevention of cancer, the method comprising the administration of an effective therapeutic amount of the nanoemulsion of the first aspect second or fourth aspect of the invention, which encapsulates one or more poorly water-soluble compounds which are pharmaceutical, to a subject in need thereof.

In view of the above, the nanoemulsion of the invention is useful as a delivery system of a poorly water-soluble pharmaceutical but also can find use in the vehiculation of non-therapeutic compounds, such as cosmetics, nutraceuticals, etc.

Thus, in a final aspect the present invention provides the use of the nanoemulsion as defined above as a drug delivery system of poorly water-soluble non-pharmaceutical compounds, such as cosmetics, nutraceuticals, etc.

## Detailed description of the invention

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the present invention, any ranges given include both the lower and the upper end-points of the range.

The present invention provides in a first a second aspect an O/W liquid nanoemulsions optionally encapsulating poorly water-soluble compounds.

The term "nanoemulsion" refers to a colloidal dispersed system comprising at least two immiscible phases, one phase dispersed in the other phase as droplet having an average size from 30 nm to 100 nm. The nanoemulsion of the invention is liquid at room temperature.

The nanoemulsion composition of the invention is an oil-in-water nanoemulsion. The terms "oil-in-water" and "O/W' have the same meaning and are used interchangeable. They refer to a nanoemulsion wherein oil is dispersed as droplets throughout the aqueous phase.

As it has been explained above, the nanoemulsion of the invention shows surprising properties due to the small average size of the oil droplets and the zeta potential.

The terms "average size" and "mean size" have the same meaning and are used interchangeable. They refer to average diameter of the droplets. The average size of these systems can be measured by standard processes known by persons skilled in the art. By "average size" and "mean size" is understood a D(n,50) droplet average size in number. The D(n,50) droplet average size is the median diameter, where 50% of the droplets are composed of droplets larger than the stated value, and 50% of the droplets are composed of droplets smaller than the stated value. In the present invention, the measurement of the average size of the droplets was performed by dynamic light scattering (DLS). DLS makes use of two common characteristics of colloids, the Tyndall effect (scattering) and the Brownian motion which cause light to be scattered at different intensities. Analyses of the time depend on the intensity fluctuations using mathematical models, allows the determination of the average size (cfr. Hassan, P. et al, "Making sense of Brownian motion: colloid characterization by dynamic light scattering", Langmuir, 2015, vol. 31, pp.3-12). The droplets are constantly moving due to Brownian motion and the relationship between the size of a droplets and its speed due to Brownian motion is defined in the Stokes-Einstein equation. As the droplets move around, the scattered light will cause intensity fluctuations. Furthermore, the signal intensity is compared at different times with itself in order to obtain the correlation function. This information can then be used to calculate the size distribution by intensity, and it can be converted to a volume or a number size distribution. Particularly, the diameter of the droplets (i.e. the mean size of the droplets) is determined using a Zetasizer Nano ZS (Malvern Instruments). In the present invention the measurement of the average size (D(n,50)) of the droplets was measured by dynamic light scattering (DLS) with Zetasizer Nano ZS (Malvern Instruments) performing the calculations explained above in the present application. In one embodiment, optionally in combination with any of the embodiments provided above or below, the sample is diluted with water (at a volume ratio 1:200) previously to the determination of the average size by Zetasizer Nano ZS. In one embodiment, optionally in combination with any of the embodiments provided above or below, the nanoemulsion is diluted with water at a volume ratio 1:75-1:125, such as 1:100, previously to the determination of the average size by Zetasizer Nano ZS. In another embodiment, optionally in combination with any of the embodiments provided above or below, the average size of the droplets forming the nanoemulsions of the invention are within the range from 35 to 90 or from 40 to 70 nm.

The term "zeta potential" is the electrical potential at the slipping plane which separates mobile fluid from fluid that remains attached to the surface. In other words, zeta potential is the potential difference between the dispersion medium and the stationary layer of fluid attached to the dispersed particle. The zeta potential is caused by the net electrical charge contained within the region bounded by the slipping plane, and also depends on the location of that plane. Thus, it is widely used for quantification of the magnitude of the charge.

The zeta potential is a key indicator of the stability of colloidal dispersions. The magnitude of the zeta potential indicates the degree of electrostatic repulsion between adjacent, similarly charged particles in a dispersion. For molecules and particles that are small enough, a high zeta potential will confer stability, i.e., the solution or dispersion will resist aggregation. When the potential is small, attractive forces may exceed this repulsion and the dispersion may break and flocculate. So, colloids with high zeta potential (negative or positive) are electrically stabilized while colloids with low zeta potentials tend to coagulate or flocculate as outlined in the table.

The zeta potential can be determined using the same Zetasizer Nano ZS (Malvern Instruments) as the one used for determining the average size of oil droplets.

In one embodiment, optionally in combination with any of the embodiments provided above or below, the zeta potential absolute value (i.e. either positive or negative) is comprised from 30 to 70 mV, particularly from 30 to 60 mV, particularly from 35 to 50 mV, particularly from 35 to 45 mV; and.

The nanoemulsion of the invention optionally includes a poorly water-soluble compound. In one embodiment of the invention, optionally in combination with any of the embodiments provided above or below, the "poorly water-soluble compound" is selected from flavours, colours, vitamins, antioxidants, antimicrobials, pesticides, herbicides, cosmetics, nutraceuticals, phytochemicals and pharmaceuticals The "solubility" is the property of a solid, liquid or gaseous chemical substance called solute to dissolve in water, which acts as solvent. The extent of solubility ranges widely, from infinitely soluble (without limit) (miscible) such as ethanol in water, to poorly soluble, such as silver chloride in water. In the context of the present invention, a compound is considered to be "poorly soluble" when its solubility is equal or lower than 1 g in 100 mL of water at 20°C. In one embodiment of the invention, optionally in combination with any of the embodiments provided above or below, the compound has a solubility comprised from 0.9 to 0.01, from 0.8 to 0.0.05, from 0.4 to 0.1 g per 100 mL of water at 20°C. In one embodiment of the invention, optionally in combination with any of the embodiments provided above or below, the compound has a solubility equal or lower than 0.9, 0.8, 0.7, 0.6, 0.5, 0.4 or 0.3 g per 100 mL of water at 20°C. In one embodiment of the invention, optionally in combination with any of the embodiments provided above or below, the compound is one of the list provided above and has a solubility comprised from 0.9 to 0.01, from 0.8 to 0.0.05, from 0.4 to 0.1 g per 100 mL of water at 20°C. In one embodiment of the invention, optionally in combination with any of the embodiments provided above or below, the compound is one of the list provided above and has a solubility equal or lower than 0.9, 0.8, 0.7, 0.6, 0.5, 0.4 or 0.3 g per 100 mL of water at 20°C.

In another embodiment of the invention, optionally in combination with any of the embodiments provided above or below, the poorly water-soluble compound is at a percentage by weight with respect to the total weight of the nanoemulsion of the invention comprised from 0.005 to 1 %, or from 0.0015 to 0.05%, or from 0.01 to 0.03%.

In another embodiment of the invention, optionally in combination with any of the embodiments provided above or below, the poorly water-soluble compound is at a percentage by weight with respect to the total weight of the nanoemulsion of the invention comprised from 0.01 to 0.05%. In another embodiment of the invention, optionally in combination with any of the embodiments provided above or below, the poorly water-soluble compound is at a percentage by weight with respect to the total weight of the nanoemulsion of the invention comprised from 0.015 to 0.025%. In another embodiment of the invention, optionally in combination with any of the embodiments provided above or below, the poorly water-soluble compound is at a percentage by weight with respect to the total weight of the nanoemulsion of the invention comprised of 0.02%.

In one embodiment of the invention, optionally in combination with any of the embodiments provided above or below, the nanoemulsion has a polydispersity index (PDI) comprised from 0.15 to 0.25, particularly from 0.17 to 0.22, particularly from 0.18 to 0.21, particularly it is selected from 0.18, 0.19, 0.20 and 0.21. The "dispersity" is a measure of the heterogeneity of size of the oil droplets in the nanoemulsion. A PDI value close to 0.2, advantageously, is indicative of a homogeneous particle size. The "PDI" value can be determined using the Zetasizer Nano ZS (Malvern Instruments), following manufacturer's instructions.

In another embodiment of the invention, optionally in combination with any of the embodiments provided above or below:

- the fatty phase is at % by weight with respect the total weight of the nanoemulsion (w/w) comprised from 9 to 11, particularly from 9.5 to 10.5; and

- the aqueous phase is at a % by weight with respect the total weight of the nanoemulsion (w/w) comprised from 75 to 85, particularly from 77 to 83%, particularly from 78 to 82%; and

The terms "percentage (%) by weight", "weight/weight %" and "w/w%" have the same meaning and are used interchangeably. They refer to the percentage of one ingredient in relation to the total weight of a composition.

In another embodiment of the invention, optionally in combination with any of the embodiments provided above or below, the nanoemulsion comprises a surfactant system having a total hydrophilic-lipophilic balance (HLB) value from 11 to 15, particularly from 11.5 to 13.5 or from 12 to 13. In another embodiment of the invention, optionally in combination with any of the embodiments provided above or below, the nanoemulsion comprises a surfactant system having a total hydrophilic-lipophilic balance (HLB) value of 12.1, 12.2, 12.3, 12.4, 12.5, 12.6, 12.7, 12.8 or 12.9.

The term "surfactant system" embraces both the embodiment wherein the nanoemulsion comprises a single surfactant or a combination of two or more surfactants, optionally in combination with a co-surfactant (enhancing the properties of the surfactant). In one embodiment, the surfactant system is made of two surfactants.

The term "surfactant", as used herein, refers to a compound that lowers the surface tension or interfacial tension between two liquids or between a liquid and a solid. Surfactants have a hydrophobic part and a hydrophilic part. Depending on the nature of the hydrophilic part the surfactants are classified as non-ionic (surfactant with a non-charged but polar hydrophilic part), anionic (when the hydrophilic part contains a negatively charged group), cationic (when the hydrophilic part contains a positively charged group) or amphoteric (when the when the hydrophilic part contains has both cationic and anionic groups).

In one embodiment of the invention, optionally in combination with any of the embodiments provided above or below, the surfactants included in the surfactant system are non-ionic surfactants. In another embodiment of the invention, optionally in combination with any of the embodiments provided above or below, the surfactant system comprises one or more, particularly 2, surfactants selected from the group consisting of phospholipids, polyoxyethylene sorbitan fatty acid derivatives, castor oil or hydrogenated castor oil ethoxylates, fatty acid ethoxylates, alcohol ethoxylates, polyoxyethylene-polyoxypropylene copolymers, block co-polymers, alkylphenol surfactants and polyglycolized medium chain triglycerides, sorbitan esters, and combinations thereof. In another embodiment of the invention, optionally in combination with any of the embodiments provided above or below, the surfactant system comprises two or more different sorbitan esters. In another embodiment of the invention, optionally in combination with any of the embodiments provided above or below, the surfactants are selected from polyoxyethylene sorbitan esters (such as Tween 20, 60 or 80) and sorbitan esters of fatty acids (such as sorbitan monostearate, sorbitan triestearate, sorbitan monolaurate, sorbitan monooleate, sorbitan trioleate, or sorbitan monopalmitate). In another embodiment of the invention, optionally in combination with any of the embodiments provided above or below, the surfactant system consist of two surfactants selected from polyoxyethylene sorbitan esters (such as Tween 20, 60 or 80) and sorbitan esters of fatty acids (such as sorbitan monostearate, sorbitan triestearate, sorbitan monolaurate, sorbitan monooleate, sorbitan trioleate, or sorbitan monopalmitate).In another embodiment of the invention, optionally in combination with any of the embodiments provided above or below, the surfactant system comprises a polyoxyethylene sorbitan ester and a sorbitan ester of a fatty acid. In another embodiment of the invention, optionally in combination with any of the embodiments provided above or below, the surfactant system consists of a mixture of polyoxyethylene sorbitan ester and a sorbitan ester of a fatty acid. In another embodiment of the invention, optionally in combination with any of the embodiments provided above or below, the surfactant system comprises surfactants selected from Tween and Span surfactants. In another embodiment of the invention, optionally in combination with any of the embodiments provided above or below, the surfactant system comprises a Tween surfactant and a Span surfactant. In another embodiment of the invention, optionally in combination with any of the embodiments provided above or below, the surfactant system consists of a mixture of a Tween and a Span surfactants In another embodiment, optionally in combination with any of the embodiments provided above or below, the surfactant system comprises Tween 80 (Polyoxyethylene sorbitan monooleate) and Span 80 (sorbitan monooleate). In another embodiment, optionally in combination with any of the embodiments provided above or below, the surfactant system consists of Tween 80 (Polyoxyethylene sorbitan monooleate) and Span 80 (sorbitan monooleate).

The "hydrophilic-lipophilic balance" (HLB) is a measure of the degree to which a surfactant is hydrophilic or lipophilic. The HLB value is determined as follows:

(i) when the surfactant system consists of one surfactant, this parameter is calculated following Equation (I):

$$HLB = 20 * M_h/M \quad (I)$$

where is the molecular mass of the hydrophilic portion of the surfactant, and M is the molecular mass of the whole surfactant; and

(ii) when the surfactant system consists of two surfactants (or a surfactant and a co-surfactant), this parameter is calculated by Equation (II):

$$HLB_{surfactant\ system} = X\ HLB_A + (1-x)\ HLB_B \quad (II)$$

wherein $HLB_A$ represents the HLB value of surfactant "A", $HLB_B$ represents the HLB value of surfactant B and X represents the fraction of surfactant A vs the fraction of surfactant B.

In another embodiment, optionally in combination with any of the embodiments provided above or below:

- the fatty phase is at % by weight with respect the total weight of the nanoemulsion (w/w) comprised from 9 to 11, particularly from 9.5 to 10.5% (w/w);
- the aqueous phase is at a % by weight with respect the total weight of the nanoemulsion (w/w) comprised from 75 to 85, particularly from 77 to 83, particularly from 78 to 82% (w/w); and

the nanoemulsion further comprises a surfactant system:

(a) which has a total hydrophilic-lipophilic balance (HLB) value from 11-15, particularly from 12-14,

(b) consists of a mixture of two surfactants, and

(c) is at a % (w/w) with respect to the total weight of the nanoemulsion comprised from 8 to 10%, particularly from 8.5 to 9.5% (w/w).

In another embodiment of the invention, optionally in combination with any of the embodiments provided above or below, the surfactant system is at a % by weigh with respect to the total weight of the composition from 8.5 to 9.5% (w/w). In another embodiment of the invention, optionally in combination with any of the embodiments provided above or below, the surfactant system is at a % by weigh with respect to the total weight of the composition of 8.1, 8.2, 8.3, 8.4, 8.5, 8.6, 8.7, 8.8, 8.9, 9.0, 9.1, 9.2, 9.3, 9.4 or 9.5% (w/w).

In another embodiment of the invention, optionally in combination with any of the embodiments provided above or below, the nanoemulsion further comprises one or more of a steric stabilizer(s) and antioxidant(s).

Any antioxidant known in the state of the art can be used in the formulation of the nanoemulsion of the invention. In one embodiment, the antioxidant is a superoxide free radical inhibitors. Illustrative non-limitative examples of suitable antioxidants are polyphenols (phenolic acids, flavonoids, anthocyanins, lignans and stilbenes), carotenoids (xantophylls and carotenes), vitamins (vitamin E and C) and phenolic compounds, among others, which can be derived from food and medicinal plants, such as fruits, vegetables, cereals, mushrooms, flowers, spices and traditional medicinal herbs, among others, or chemically synthesized. In one embodiment the antioxidant is a vitamin, such as a vitamin E, such as α-tocopherol.

In another embodiment of the invention, optionally in combination with any of the embodiments provided above or below, the antioxidant is at a % by weight with respect to the total weight of the nanoemulsion comprised from 0 to 5% (w/w), 0.5 to 2% (w/w), particularly 1% (w/w).

In the present invention, the term "steric stabilizer" means a compound that adsorbs at the droplet's surface, thus preventing other particles from approaching because of steric repulsion forces. Steric stabilizers have long been used to achieve greater stability of colloidal particles or to prevent capture by phagocytic cells. In one embodiment of the first aspect of the invention, optionally in combination with any of the embodiments provided above or below, the steric stabilizer has a molecular weight of at least 10 kDa in order to counteract the van der Waals attraction and to impart nanoparticle stability. When the steric stabilizer is a polymer, then the term "molecular weight", refers to the weight average molecular weight ("Mw"). Illustrative non-limitative examples of suitable steric stabilizers are poloxamers (188, 338, 407), polyvinyl alcohol (PVA), and polyethylene glycol 2000 (PEG 2000), among others. In the case of poloxamers, they further act as a molecular spacer, providing a long range repulsion between the colloidal particles.

In one embodiment of the first aspect of the invention, the steric stabilizer is a poloxamer, such as a poloxamer 188. These polymers are safe because they are commonly used in industrial applications, cosmetics, and pharmaceuticals. They have also been evaluated for various drug delivery applications and were shown to sensitize drug-resistant cancers to chemotherapy. In bioprocess applications, poloxamers are used in cell culture media for their cell cushioning effects because their addition leads to less stressful shear conditions for cells in reactors.

In another embodiment of the invention, optionally in combination with any of the embodiments provided above or below, the steric stabilizer is at a % by weight with respect to the total weight of the nanoemulsion comprised from 0 to 0.5% (w/w), particularly from 0.01 to 0.2% (w/w), particularly from 0.05 to 0.15% (w/w), particularly of 0.1% (w/w).

In one embodiment of the invention, optionally in combination with any of the embodiments provided above or below, the fatty phase (or oil) comprises a long chain fatty acid, particularly a long chain triglyceride having from 14 to 22 carbon atoms.

The term "oil" is used herein in a general sense to identify a wide class of substances typically unctuous, viscous, and liquid at room temperature. Oil as here in defined can be from animal, mineral, vegetable or synthetic origin.

The term "oil component" refers to oil, or a combination of multiple oils in a colloidal dispersion. The term "room temperature" refers to a temperature of the environment, without heating or cooling, and is generally from 20 °C to 25 °C.

Illustrative non-limitative examples of useful long chain triglyceride in the context of the invention are selected from the group consisting of animal, vegetable, flavor oils, essential oils, water-insoluble vitamins, isopropyl stearate, butyl stearate, octyl palmitate, cetyl palmitate, tridecyl behenate, diisopropyl adipate, dioctyl sebacate, menthyl anthranilate, cetyl octanoate, octyl salicylate, isopropyl myristate, ketoles of neopentylglycol dicaprate, decyl oleate, $C_{12}$-$C_{15}$ alkyl lactates, cetyl lactate, cetyl lactate, cetyl lactate lactate, isocetyl stearoyl stearate, octyldodecyl stearoyl stearate, argan oil, rapeseed oil, chili oil, coconut oil, corn oil, cotton oil, flax oil, grape seed oil, mustard oil, olive oil, palm oil, fractionated palm oil, peanut oil, castor oil, a Pine seed oil, poppy seed oil, pumpkin seed oil, rice bran oil, safflower, tea oil, truffle oil, vegetable oil, apricot oil, jojoba oil, macadamia oil, oil wheat germ, almond oil, soybean oil, sesame oil, hazelnut oil, sunflower oil, hemp oil, bois oil, Kukui nut oil, avocado oil, walnut oil, fish oil, berry oil, allspice oil, juniper oil, seed oil, almond seed oil, anise seed oil, celery seed oil, cumin seed oil, nutmeg oil, basil leaf oil, bay leaf oil, cinnamon leaf oil, common sage leaf oil, eucalyptus leaf oil, eucalyptus leaf oil, lemon leaf oil, melaleuca leaf oil, oregano oil, patchouli leaf oil, peppermint leaf oil, pine needle oil, rosemary leaf oil, spearmint oil, tea tree leaf oil, thyme leaf oil, Canada tea leaf oil, flower oil, chamomile oil, sage oil, clove oil, geranium flower oil, flower oil hyssop, jasmine flower oil, lavender flower oil, mauka flower oil, marjoram flower oil, orange flower oil, rose flower oil, ylang-ylang flower oil, bark oil, Cassia Bark Oil, Cinnamon Bark Oil, Sassafras Bark Oil, Wood Oil, Camphor Wood Oil, Cedar Wood Oil, Rosewood Oil, Sandalwood Oil, Ginger Wood Oil, resin oil, castor oil, myrrh oil, skin oil, Bergamo skin oil, lemon peel oil, lime peel oil, orange peel oil, tangerine peel oil, root oil, valerian oil, oleic acid, linoleic acid, oleyl alcohol, isostearyl alcohol, oleate of ethyl, Miglyol®, Labrafil®, Labrafac®, Rylo®, Peceol® and Maisine®, synthetic or semisynthetic derivatives thereof and combinations thereof.

In one embodiment of the invention, optionally in combination with any of the embodiments provided above or below, the fatty phase comprises one or more long chain triglycerides which are polyoxyl glycerides. In the present invention, the term "polyoxyl glyceride" (also known as "macrogol glyceride") means that the long chain triglyceride has one or more oxyl units of formula -[($C_1$-$C_{20}$)alkylene-O]-, such as -[$CH_2$-$CH_2$-O]- units. In one embodiment, optionally in combination with any of the embodiments provided above or below, the fatty phase comprises macrogolglycerides, such as an oleyl macrogols, such as oleoyl polyoxyl-6 glycerides (Labrafil M 1944®).

In another embodiment of the invention, the nanoemulsion is one selected from the group consisting of:

(a) - oleoyl polyoxyl-6 glyceride (such as Labrafil M 1944®): 9.5-10.5 % (w/w);

- water: 70-85% (w/w);
- surfactant system having a HLB value from 12.5 to 13.00, the system being at a % by weight comprised from 8.5 to 9.5% (w/w); and
- a poorly water-soluble compound at a % by weight comprised from 0.005 to 0.1% or from 0.01 to 0.05% or from 0.015 to 0.025% (w/w);

(b) - oleoyl polyoxyl-6 glyceride (such as Labrafil M 1944®): 9.5-10.5% (w/w);

- water: 70-85% % (w/w);
- the surfactant system having a HLB value from 12.5 to 13.0, comprising a polyoxyethylene sorbitan fatty acid derivative and a sorbitan ester of a fatty acid, and the surfactant system being at a % by weight comprised from 8.5 to 9.5; and
- optionally a poorly water-soluble compound at a % by weight comprised from 0.005 to 0.1% or from 0.01 to 0.05% or from 0.015 to 0.025% (w/w);

(c) - oleoyl polyoxyl-6 glyceride (such as Labrafil M 1944®): 9.5-10.5% (w/w);

- water: 70-85% (w/w);
- the surfactant system having a HLB value from 12.5 to 13.0, comprising polyoxyethylene sorbitan monooleate (Tween 80) and sorbitan monooleate (Span 80), and the surfactant system being at a % by weight comprised from 8.5 to 9.5% (w/w); and
- optionally a poorly water-soluble compound at a % by weight comprised from 0.005 to 0.1% or from 0.01 to 0.05% or from 0.015 to 0.025% (w/w);

provided that the sum of the components does not exceed the 100%.

In still another embodiment of the first aspect of the invention, optionally in combination with any of the embodiments provided above or below, the nanoemulsion comprises:

(d) oleoyl polyoxyl-6 glyceride (such as Labrafil M 1944 $^®$): 9.5-10.5% (w/w);
water: 75-85% (w/w);
surfactant system with a HLB value from 12.5 to 13: 8-10 % (w/w);
steric stabilizer(s): 0.05-0.2 % (w/w);
antioxidant(s): 0.5-1.5%; and
optionally a poorly water-soluble compound (such as sulforaphane): 0.015-0.025%, such as 0.02% (w/w); or, alternatively,

(e) oleoyl polyoxyl-6 glyceride (such as Labrafil M 1944 $^®$): 9.5-10.5% (w/w);
water: 75-85% (w/w);
surfactant system consisting of polyoxyethylene sorbitan monooleate (Tween 80) and sorbitan monooleate (Span 80), the system a HLB value from 12.8 to 12.9:

8-10 % (w/w)
poloxamer(s): 0.05-0.2 % (w/w);
vitamin(s), such as $\alpha$-tocopherol: 0.5-1.5% (w/w); and
optionally a poorly water-soluble compound (such as sulforaphane): 0.015-0.025%, such as 0.02% (w/w).

In another embodiment of the invention, optionally in combination with any of the embodiments provided above or below, the poorly water-soluble compound is a pharmaceutical.

Illustrative non-limitative examples of poorly water-soluble compounds are sulfur-containing compounds, polyphenol-based compounds, and glucosinolates, among others.

Sulfur containing compounds like glucosinolates and their derivatives like isothiocyanates has been proved to possess several health benefits like anti-cancer activity, to decrease the risk of cardiovascular diseases, ability to reduce LDL and prevention of metabolic disorders, Alzheimer's disease and reduction of obesity (cfr. Thirumdas, R. et al. "Health benefits of dietary glucosinolates and its derived isothiocyanates", Indian Food Industry, 2017, 36(2), 31-36). In another embodiment of the invention, optionally in combination with any of the embodiments provided above or below, the poorly water-soluble compound is an isothiocyanate-based compound such as sulforaphane, allyl isothiocyanate, phenethyl isothiocyanate, and benzyl isothiocyanate, particularly sulforaphane.

The main groups of polyphenols are: phenolic acids (derived from hydroxybenzoic acid: syringic, gallic, vainillic acids; or hydroxycinnamic acid: caffeic, ferulic, sinapic, p-coumaric acids), stilbenes (resveratrol), lignans (secoisolariciresinol, matairesinol, lariciresinol, pinoresinol, medioresinol, syringaresinol, enterodiol), tannins (gallic acid, chlorogenic acid) phenolic alcohols (eugenol, thymol, n-hexylresorcinol, tyrosol) and flavonoids (hesperidin, naringenin, eriodictyol, quercetin, kaempferol, epicatechin, cyanidin, malvidin, peonidin, genistein, daidzein, glycetin). The regular consumption of polyphenols may help decrease the incidence of cardiovascular diseases, colon cancer, liver disorders, obesity, diabetes. Of the family of polyphenols, flavonoids have received the most attention. Quercetin, kaempferol, catechins, resveratrol, rutin and their derivatives are known for their health benefits end the prevention of various diseases, especially cancer, cardiovascular diseases, diabetes, obesity, liver disorder and infectious diseases. Growing evidence also indicates that polyphenols may prevent neurodegenerative diseases such as Alzheimer's and Parkinson's diseases.

In another embodiment of the invention, optionally in combination with any of the embodiments provided above or below, the poorly water-soluble compound is a pharmaceutical or cosmetical agent, and the nanoemulsion further includes one or more pharmaceutically or cosmetically acceptable excipients. In another embodiment of the invention, optionally in combination with any of the embodiments provided above or below, the poorly water-soluble pharmaceutical agent is a sulfur-containing compound, such as an isothiocyanate. In another embodiment of the invention, optionally in combination with any of the embodiments provided above or below, the poorly water-soluble pharmaceutical agent is sulforaphane.

The term "acceptable excipients or carriers" refers to acceptable material, composition or vehicle, which include without limitation pH adjusting agents, preservatives, antioxidants, chelating agents, stabilizers, viscosizing agents, adhesive polymers, penetration enhancers and tonicity agents. Each component must be acceptable in the sense of being compatible with the other ingredients of the composition. It must also be suitable for use in contact with the tissue or organ of humans and animals without excessive toxicity, irritation, allergic response, immunogenicity or other problems or complications commensurate with a reasonable benefit/risk ratio.

In an embodiment, the nanoemulsion of the invention further comprises one or more pH adjusting agents. The term "pH adjusting agent" refers to acids or bases or their mixtures that can be used to adjust the pH of the finished product to the desired level, without affecting the stability of the emulsion. In an embodiment, the nanoemulsion of the invention further comprises a pH adjusting agent selected from the group consisting of lactic acid and salts thereof (such as sodium lactate, potassium lactate and calcium lactate), citric acid and salts thereof (such as sodium citrate, potassium citrate, calcium citrate, lithium citrate, trisodium citrate and disodium hydrogen citrate), tartaric acid and salts thereof (such as sodium tartrate potassium tartrate, calcium tartrate and lithium tartrate), acetic acid and salts thereof (such as sodium acetate, potassium acetate and calcium acetate), hydrochloric acid, boric acid and salts thereof (sodium borate), sulphuric acid and salts thereof (such as sodium sulphate and potassium sulphate), nitric acid, , phosphoric acid and salts thereof (such as sodium dihydrogen phosphate, sodium monohydrogen phosphate, potassium dihidrogen phosphate lithium phosphate, potassium phosphate and calcium phosphate), carbonic acid and salts thereof (such as sodium carbonate, sodium hydrogen carbonate and potassium hydrogen carbonate), maleic acid and salts thereof (lithium maleate, sodium maleate, potassium maleate and calcium maleate), succinic acid and salts thereof (lithium succinate, sodium succinate, potassium succinate and calcium succinate), sodium hydroxide, potassium hydroxide, triethanolamine, diisopropanolamine, ammonia, tris(hydroxymethyl)aminomethane, tris(hydroxymethyl)aminomethane hydrochloride, and mixtures thereof. In an embodiment, the pH adjusting agent is selected from the group consisting of tris(hydroxymethyl)aminomethane, tris(hydroxymethyl)aminomethane hydrochloride, potassium dihydrogen phosphate, disodium hydrogen phosphate and mixtures thereof.

In an embodiment, the nanoemulsion of the invention further comprises a pH adjusting agent selected from the group consisting of acetic acid, boric acid, sorbic acid, citric acid, phosphoric acid, sodium phosphate, dibasic sodium phosphate, monobasic sodium phosphate, potassium dihydrogen phosphate and salts thereof, hydrochloric acid, sodium hydroxide, sodium thiosulfate, sodium sulfite, sodium sulphate, tris(hydroxymethyl)aminomethane, tris(hydroxymethyl)aminomethane hydrochloride, sodium hydrogen carbonate, sodium borate, sodium acetate, sodium bisulphate, sodium benzoate, sodium citrate and mixtures thereof.

In an embodiment, the nanoemulsion of the invention further comprises one or more bioadhesive polymers. The term "bioadhesive polymers" refers to a substance which can increase residence time of the compositions of the invention. Examples of bioadhesive polymers appropriate for the present invention include polyvinylpirrolidones, such as Povidone K 17, Povidone K25, Povidone K 30 and Povidone K 90F; polyvinyl alcohol; xanthan gum; guar gum; welan gum; gellan gum; tragacanth gum; ceratonia gum; agar; methylcellulose; ethylcellulose; hydroxyethyl cellulose; hydroxyethylmethyl cellulose; hydroxypropyl cellulose; hydroxypropylmethyl cellulose; hydroxypropylmethyl cellulose phthalate; hydroxypropylmethyl cellulose acetate succinate; sodium carboxymethylcellulose; calcium carboxymethylcellulose; polyethylene glycol; glycerine; carrageenan; alginic acid; sodium alginate; potassium alginate; propylene glycol alginate; hyaluronic acid; sodium hyaluronate; poly(acrylic acid) derivatives such as carbomer and polycarbol; poloxamers; chitosan and chitosan derivatives; vinyl methyl ether/maleic anhydride copolymers; maltodextrin; and mixtures thereof. In an embodiment, the compositions of the invention comprise polyvinylpyrrolidone as bioadhesive polymer. In an embodiment, the bioadhesive polymer is present in an amount of from 0.01 % to 15 % by weight with respect to the total weight of the compositions.

In an embodiment, the nanoemulsion of the invention further comprises one or more preservatives. The term "preservative" refers to a compound that preserve from microbial and/or fungal contaminations. Examples of preservatives appropriate for the present invention include but is not limited to benzalkonium chloride, cetalkonium chloride, bezethonium chloride, chlorhexidine, benzyl alcohol, chlorobutanol, 2-phenylethanol, propylparaben, methylparaben, phenylmercuric acetate, phenylmercuric borate, sodim dehydroacetate, sorbic acid phenylmercuric nitrate, cetyl pyridinium chloride, cetrimonium bromide, benzyl bromide, sodium perborate, thimerosal and mixture thereof. The amount of the preservative in the nanoemulsion of the present invention is comprised from 0 % to 1 % by weight.

In an embodiment, the nanoemulsion of the invention further comprises one or more tonicity agent. The term "tonicity agent" refers to a compound that can be used for adjusting the osmolality of the nanoemulsion. In an embodiment, the tonicity agent is selected from the group consisting of sodium chloride, potassium chloride, calcium chloride, sodium phosphate, potassium phosphate, sodium hydrogen carbonate, calcium carbonate, sodium lactate, sorbitol, mannitol, xylitol, dextrose, polyethylene glycol, propylene glycol, dextran, and mixture thereof; preferably glycerin. The amount of the tonicity agent in the nanoemulsion of the present invention is comprised from 0 % to 15 % by weight.

In an embodiment, the nanoemulsion of the invention further comprises one or more chelating agent. The term "chelating agent" and "chelant" have the same meaning and are used interchangeable. They refer to a compound that is capable of complexing ions.

Examples of chelating agents are citric acid, in particular citric acid monohydrate, EDTA (ethylenediaminetetraacetic acid) and its salts, such as dipotassium EDTA, disodium EDTA, calcium disodium EDTA, sodium EDTA and trisodium EDTA, fumaric acid, malic acid and maltol. In an embodiment, the chelating agent is selected from the group consisting of sodium edetate, citric acid, and salt and mixture thereof. The amount of the chelating agent in the nanoemulsion of the present invention is comprised from 0 % to 2 % by weight.

The term "penetration enhancer", as used herein, refers to a substance which enhances drug penetration. Examples of penetration enhancers are surfactants such as sorbitan monolaurate, sorbitan monopalmitate, sorbitan trioleate, polyoxyethylene 20 sorbitan monolaurate, polyoxyethylene 20 sorbitan monopalmitate, polyoxyethylene 5 sorbitan monooleate, polyoxyethylene 20 sorbitan trioleate, polyoxyethylene 9 lauryl ether, polyoxyethylene 23 lauryl ether, polyoxyethylene 20 cetyl ether, polyoxyethylene 20 oleyl ether, polyethylene glycol octadecyl ether, polyoxyethylene 40 stearate, polyoxyethylene 50 stearate, palmitoyl carnitine, sodium caprate, sodium dodecyl sulfate, bile acids such as deoxycholic acid, taurocholic acid, taurodeoxycholic acid, urodeoxycholic acid, and tauroursodeoxycholic acid, fatty acids such as capric acid, caprylic and oleic acid, lauralkonium chloride, benzalkonium chloride, cetalkonium chloride, cetrimonium bromide , chlorhexidine digluconate, benzyl alcohol, chlorbutanol, 2-phenylethanol, paraben, propyl paraben and methyl paraben, EDTA, 1-dodecylazacycloheptan-2-one (Azone), hexamethylene lauramide, hexamethylene octanamide, decylmethylsulfoxide, saponin, cyclodextrins, pz-peptide, α-amino acid, cetylpyridinium chloride, cytochalasins, ionophores or mixtures thereof. The amount of the penetration enhancer in the nanoemulsion of the present invention is comprised from 0.01 % to 10 % by weight.

In a third aspect the present invention provides a process for preparing the nanoemulsion of the invention.

In one embodiment of the third aspect of the invention, the process comprises: (a) the fatty phase, optionally including the poorly water-soluble compound(s), the aqueous phase and the surfactant system are high-speed blended under agitation, from 10000 to 20000 rpm, particularly from 12000 to 18000, particularly 16000 rpm, at room temperature, for example using a Ultraturrax blender or homogenizer; and (b) subjecting the resulting blend to ultrasonication, particularly in an ice bath.

In another embodiment of the process of the second aspect of the invention, optionally in combination with any of the embodiments provided above or below, wherein one or more steric stabilizers and/or antioxidants are also added to the other components.

The invention also provides a nanoemulsion obtainable by the process of the invention.

The term "obtainable" and "obtained" have the same meaning and are used interchangeably. In any case, the expression "obtainable" encompasses the term "obtained".

In a fifth aspect the present invention provides a nutraceutical composition comprising the nanoemulsion of the invention.

In one embodiment of the fifth aspect of the invention, the nutraceutical composition comprises a nanoemulsion as defined above, wherein the poorly water-soluble compound(s) are nutraceuticals. In the present invention, the term "nutraceutical" or 'bioceutical' means a natural or synthesized substance that provides at least a perceived health or medical benefit. In this embodiment, the nutraceutical composition could be easily prepared following the process of the second aspect of the invention, firstly mixing the nutraceutical compound with the oil phase, and then with the aqueous phase, surfactant and the antioxidants and steric stabilizers, if required.

In a sixth aspect the present invention provides a functional food product comprising the nanoemulsion of the invention.

The term "functional food product" refers to processed foods having disease-preventing and/or health-promoting benefits in addition to their nutritive value. Functional foods do not cure or prevent illnesses by themselves and are not essential to the diet. Functional foods must be viewed in the context of a healthy diet to exert their potential interest "Nutraceutical" is a substance that provides medical or health benefits, encompassing prevention and treatment of disease.

In a seventh aspect the invention provides the nanoemulsion of the invention for use as a delivery system of poorly water-soluble pharmaceutical compounds.

Illustrative non-limitative examples of poorly water -soluble pharmaceutical compounds have been provided above.

In one embodiment of the seventh aspect of the invention, the nanoemulsion is as defined in any of the embodiments provided above and encapsulates one poorly water-soluble pharmaceutical compound selected from the above lists. In another embodiment of the seventh aspect of the invention, the nanoemulsion is as defined in any of the embodiments provided above and encapsulates one poorly water-soluble pharmaceutical compound which is an isocyanate-base compound as defined above, particularly sulforaphane.

In one embodiment of the eighth and ninth aspects of the invention, the nanoemulsion is as defined in any of the embodiments provided above and encapsulates a single poorly water-soluble pharmaceutical compound. Illustrative non-limitative examples of these kind of compounds have already been provided above.

In another embodiment of the eighth and ninth aspects of the invention, the nanoemulsion is as defined in any of the embodiments provided above, encapsulates an isocyanate-based pharmaceutical compound, as defined above. In another embodiment of the eighth and ninth aspects of the invention, the nanoemulsion is as defined in any of the embodiments provided above, encapsulates an isocyanate-based pharmaceutical compound, as defined above, and the disease to be treated is cancer. In another embodiment of the eighth aspect of the invention, the nanoemulsion is as defined in any of the embodiments provided above, and encapsulates sulforaphane. In another embodiment of the eighth aspect of the invention, the nanoemulsion is as defined in any of the embodiments provided above, encapsulates sulforaphane and the disease is cancer, particularly skin or gastrointestinal cancer. The gastrointestinal cancer can be selected from aesophageal cancer, gastric cancer, pancreatic cancer, and intestine, such as colon, cancer.

Finally, in the use of the nanoemulsion as defined in the present invention as a carrier for the vehiculation of poorly water-soluble compounds other than poorly water-soluble pharmaceutical compounds. All the embodiments provided above regarding the nanoemulsion are also embodiments of the use as a drug delivery system. In one embodiment, the poorly water-soluble compound other than a pharmaceutical compound is selected from a nutraceutical or cosmetical agent. Illustrative non-limitative examples of nutraceuticals are vitamins, polyunsaturated fatty acids, phenolic compounds, carotenoids, stilbenes, of cosmeticals are unsaturated fatty acids, hyaluronic acid, peptides, vitamins, antioxidants, titanium dioxide.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

#### 1. Materials

Labrafil M 1944 CS (Oleoyl polyoxyl-6 glycerides), long-chain triglycerides were received as free sample from Gateffosé (Lyon, France). Tween 80 (polysorbate 80) and Span 80 were a gift from Croda Ibérica S.A. (Barcelona, Spain). D,L-Sulforaphane and $\alpha$-tocopherol was purchased from Sigma-Aldrich (Madrid, Spain).

3T3 mouse fibroblast (ECACC86110401), Caco-2 human colorectal epithelial cells (ATCC® HTB-37™) and HaCaT human keratinocytes (CLS 300493).

MTT (3-(4,5-dimethylthiazol-2-yl)-2,5-diphenyltetrazolium bromide) (Sigma, USA).

Carboxy-H2DFFDA, Invitrogen (Fisher Scientific, Spain).

#### 2. Nanoemulsion formulations

2.1. Nanoemulsion formulation "A"

OIL PHASE (10,1%): Labrafil M 1944 CS
AQUEOUS PHASE (80.8%): water
SURFACTANTS (9.1%): Tween 80/Span 80 (HLB 12.9)
STERIC STABILIZER (0.1%): Kolliphor 188
ANTIOXIDANT: $\alpha$-tocopherol (1%)

2.2. Nanoemulsion formulation "B" (with a poorly-soluble compound (sulforaphane))

OIL PHASE (10.1%): Labrafil M 1944 CS
AQUEOUS PHASE (80.8%): water
SURFACTANTS (9.1%): Tween 80/Span 80 (HLB 12.9)
STERIC STABILIZER (0.1%): Kolliphor 188
ANTIOXIDANT: $\alpha$-tocopherol (1%)
SULFORAPHANE: 0.02 %

#### 3. Preparation of nanoemulsion "A".

3.1. Option 1

The mixture of oil, surfactants and active compound was stirred for about 5 min, before adding the aqueous phase dropwise and continue stirring for 5 minutes more. After, the mixture was subjected to a high-speed blender (Ultraturrax T25, IKA) at 16.000 rpm for 2 minutes at room temperature to prepare the nanoemulsion.

3.2. Option 2

The same protocol as the one of 3.1. was followed but, after preparing the coarse emulsion, this emulsion was treated by ultrasonication in an ice-bath (Sonics Materials VC-750-220, Sonics & Materials, Inc. Newtown, USA). Conditions: Amplitude: 40%; Sonotrode: 6 mm; time: 10 min (pulse ON: 10 s, pulse OFF: 3s).

In all cases a blank nanoemulsion was obtained.

## 4. Determination of Cell Viability and cytotoxicity of nanoemulsion A

In this task, the *in vitro* toxicity of the nanoemulsion A (option 2) was evaluated prior to encapsulation of the selected bioactive (sulforaphane).

The cell lines used in the present study were 3T3 mouse fibroblast (ECACC86110401), Caco-2 human colorectal epithelial cells (ATCC® HTB-37™) and HaCaT human keratinocytes (CLS 300493). All cell lines were routinely grown as a monolayer in tissue culture grade flasks in a humidified atmosphere with $5 \pm 1\%$ $CO_2$/air. When the cell lines exceeded 70% confluence (but less than 90%), they were sub-cultured on new culture flasks. At least 24 hours before the performance of the assays, the cells were tripsynized, counted and cell suspensions of 100.000 cells/well for 24 well plates or 10.000 cells/well for 96-well plates were prepared. An appropriate volume of the cell suspensions was dispensed in 96 wells plates (MTT) and 24 wells plates (ROS production and annexin IP studies) respectively. Plates were pre-incubated at $37 \pm 1$°C, $5 \pm 1\%$ $CO_2$/air for 24 hours in a humidified atmosphere.

3T3, Caco-2 and HaCaT cells were treated with serial dilutions of the nanoemulsion for 24 hours. A range-finding experiment using 1:2 dilutions was performed to determine the 4 concentrations to be used starting from 5 % (vol/vol). After incubation with the test item, cells were washed twice with PBS and dyed with MTT (Sigma, USA). Plates were incubated at 37°C for 2-3 hours and at the end of the incubation time media was removed and 100 μl of DMSO was then added. 15 minutes incubation in the dark was followed by gentle agitation until a homogeneous solution was obtained. Absorbance was read at 540 nm in a plate reader. The percentage of cell viability was calculated from the obtained results. The corresponding standard deviation was calculated for three independent experiments and six replicates for each experimental point. C represents a negative control, cells without any treatment

The results are provided in Table 1 below:

**TABLE 1**

| | | | | % vol/vol Nanoemulsion | | |
| --- | --- | --- | --- | --- | --- |
| | | | Control | 1,25 | 0,625 |
| Values cyotoxicity relative to Control | 3T3 | Average | 100,000 | 95,088 | 100,115 |
| | | Desvest | 3,596 | 4,631 | 5,351 |
| | Caco-2 | Average | 100,000 | 76,409 | 80,484 |
| | | Desvest | 3,362 | 4,556 | 4,081 |
| | HaCaT | Average | 100,000 | 56,680 | 63,838 |
| | | Desvest | 1,309 | 3,554 | 3,878 |

Intracellular ROS levels were assessed by using the oxidant-sensitive fluorescent probe Carboxy-H2DFFDA (Carboxy-H2DFFDA, Invitrogen). This method is based on the passive cell diffusion of this probe and on the intracellular removal of its acetate groups by intracellular esterases and oxidation (by the activity of reactive oxygen species, ROS) to the yield a highly green-fluorescent form. All cell lines were exposed to different concentrations of the nanoemulsion A for 18 hours and fluorescence was measured by Flow cytometry (Cytomics FC500 Flow Cytometer). 10.000 events were acquired for each replicate (Excitation 488 nm, emission 425 nm). Three independent assays were performed with three replicates each. The values (percentage of positive cells) for the nanoemulsion treatment were compared to the negative control, cells without any treatment. Furthermore, cells treated with 0.5mM $H_2O_2$ for 30 minutes were included as a positive control.

As a result of these two tests, it was found that the optimal subtoxic concentration was 1%. Therefore, this concentration was selected as the optimal application dose for the following pharmaceutical tests.

## 5. Preparation of nanoemulsion "B"

The next step consisted of incorporating the active compound (sulforaphane) into the nanoemulsion "A".

From this data, and considering also that the effective concentration of sulforaphane determined ranged between 6 and 25 $\mu$M, the necessary amount of sulforaphane to be incorporated into the nanoemulsion was calculated so that, in a nanoemulsion solution at 1 %, the amount of sulforaphane intended to be encapsulated was 12.5 $\mu$M (which represented a 0.02% w/w of the total weight of the emulsion). This amount of active compound was incorporated into the oil phase, proceeding to obtain the nanoemulsion under the same conditions as those described above under any of the options 1 to 2, above.

## 6. Characterization of the nanoemulsions

6.1. Particle size polydispersity index (PDI), zeta potential, average diameter and polydispersity index were measured on an intensity basis by means of photon correlation spectroscopy using Zetasizer Nano (Malvern), at 23°C after suitable dilution (1:100) in MilliQ water. Zeta potential was assessed by determining the particle electrophoretic light scattering using the same equipment, and following manufacturer's instructions.
Thus, the values obtained for the selected formulation were:

Z-average size (nm): 55.50 ($\pm$ 1.66)

Polydispersity Index (PDI): 0.195 ($\pm$ 0.003)

Z potential of the analyzed nanoemulsion: -40.8 ($\pm$ 1.08)

The values obtained with the nanoemulsion formulation already including the poorly soluble drug (i.e. sulforaphane) were the following:

Z-average size (nm): 49.70 ($\pm$ 1.72)

Polydispersity Index (PDI): 0.177 ($\pm$ 0.002)

Z potential of the analyzed nanoemulsion: -42.3 ($\pm$ 1.21)

A small particle size such as that obtained for this nanoemulsion, favours its stability against flocculation or coalescence phenomena.
On the other hand, a PDI value close to 0.2 was indicative of a homogeneous particle size. The pH of the nanoemulsion has been measured with a conventional pH and its value was 4.61 ($\pm$ 0.03).
The zeta potential is a method of measuring the surface charge of particles and is used to predict the stability of dispersion. The zeta potential of $\pm$ 30mV is enough to guarantee the physical stability of the nanoemulsion. These negative potential zeta values contribute to the storage stability of the nanoemulsion since the repulsive forces exceed the attractive forces between the drops and this drop-by-drop repulsion prevents coagulation and / or coalescence of the dispersed phase of the emulsions. This strong stability was confirmed by subjecting freshly prepared nanoemulsion to a centrifugation process for 30 min and 1500 x g, at 5°C and 23°C. In both cases, it was observed that both the particle size and the polydispersity index did not vary significantly, indicating that the nanoemulsion is stable.

### 6.2. Sulforaphane encapsulation and loading capacity

For determining the amount of sulforaphane finally entrapped in the formulation ("EE"), weighed amount of freshly prepared SFN-loaded nanoemulsion as disclosed above was centrifuged at 10,000 x g for 30 minutes to remove excess SFN. The precipitate was separated and extracted with 1 ml of pure ethanol with vortexing for 5 minutes.
Sulforaphane content was estimated by analyzing the compound spectrophotometrically at $\lambda$max (245 nm) according to a sulforaphane (SFN) calibration curve generated at various concentrations dissolved in ethanol, at 245 nm using a UV-vis spectrophotometer (y = 0.0016x + 0.0935, R2 = 0.9975).
The entrapment efficiency (EE):

$$EE = \text{drug content in the product obtained (mg)/total amount of drug added (mg)} \times 100$$

and
It was found that the encapsulation efficiency was remarkably high, being around 93%. This means that the system is highly efficient in encapsulating the desired dose in the capsules.

6.3. Stability of the nanoemulsions

A stability test of nanoemulsion "B" was carried out at three temperatures: 4 and 23°C for 15 days. Both at room temperature and in refrigeration, the nanoemulsion showed insignificant little variation in particle size and polydispersity index with respect to the initial values (data not shown).

Regarding the possible negative effect on the encapsulated SFN, after the 15 days 0.1 mL of the nanoemulsion was transferred to 5 mL of ethanol. The ethanol solution was passed through a syringe filter (0.45mm) and its absorbance measured at 245nm (as disclosed above). A relatively lower SFN content than the initial one was observed (88% for the one stored at 4 ° C and 82% for the one stored at room temperature), which indicates that the nanoemulsions maintained both stability and encapsulation efficiency relatively good.

7. Activity test

**Determination of cell antiproliferation efficacy**

3T3, Caco-2 and HaCaT cells were treated with serial dilutions of the sulforaphane loaded nanoemulsion or with equal amounts of sulforaphane for 72 hours, a negative control condition, any treatment added, was also included. After incubation with test items, cells were washed twice with PBS and dyed with MTT (Sigma, USA). Plates were incubated at 37°C for 2-3 hours and at the end of the incubation time media was removed and 100 $\mu$l of DMSO was then added. 15 minutes incubation in the dark was followed by gentle agitation until a homogeneous solution was obtained. Absorbance was read at 540 nm in a plate reader. The percentage of proliferation (cell viability) was calculated from the obtained results with respect the negative control. The corresponding standard deviation was calculated for three independent experiments and six replicates for each experimental point.

The results are provided in Table 2 below.

It was found that the nanoemulsion, at any of the concentrations tested, presented greater anti-proliferative efficacy in all the cell lines tested compared to free sulforaphane, thus exhibiting its potential as a treatment against cancer.

**Apoptosis/necrosis induction**

Potential apoptosis and necrosis induction was studied in the cell lines using Annexin V conjugated with Alexa Fluor 488 (Molecular Probes) and Propidium Iodide (PI, Sigma) fluorochromes by Flow Cytometry, to measure early apoptotic event and necrosis respectively, after exposure to different concentrations of sulforaphane or sulforaphane-loaded nanoemulsion suspensions. HaCaT, and 3T3 cells were seeded in 24 well plates at a density of 100.000 cells/well. 24 hours later cells were exposed to test items for further 24 hours. After the exposure, 5 $\mu$L of the annexin V conjugates were added to each 100 $\mu$L of cell suspension replicate (minimum of 10.000 cell/100$\mu$L) and incubated at room temperature for 15 minutes. After incubation, cells were washed and submitted to flow cytometry acquisition. Immediately before acquisition, the stained cells were contrasted with PI (5 $\mu$g/ml) to detect necrotic cells and analysed by Cytomics FC500 Flow Cytometer. Three independent experiments and at least three replicates for each experimental point were carried out. Each experiment included a negative control (untreated cells). Results were expressed as the percentage of Annexin-V and Propidium Iodide positive cells and are provided in Table 3 below. The results obtained in 3T3 fibroblasts and in the cell line HaCaT indicated that, after 24 hours of exposure, the sulforaphane-loaded nanoemulsion conferred an increased apoptosis compared to free sulforaphane.

Table 2

| | | | C- | 12,5 | 6,25 | 3,13 | 1,56 | 0,78 | 0,39 | 0,20 |
|---|---|---|---|---|---|---|---|---|---|---|
| Caco-2 | Sulphoraphane | Average | 100,000 | 105,359 | 106,292 | 100,611 | 98,991 | 99,045 | 99,836 | 105,284 |
| | | Desvest | 3,768 | 3,363 | 3,572 | 3,852 | 5,170 | 6,065 | 6,327 | 7,602 |
| | Nanoemulsion + Sulphoraphane | Average | 100,000 | 74,101 | 74,096 | 72,068 | 71,784 | 76,069 | 83,471 | 89,322 |
| | | Desvest | 2,409 | 5,986 | 3,471 | 3,629 | 3,388 | 4,530 | 6,435 | 8,000 |
| HaCaT | Sulphoraphane | Average | 100,000 | 80,079 | 90,107 | 91,413 | 91,238 | 90,924 | 92,894 | 96,106 |
| | | Desvest | 2,796 | 4,180 | 8,440 | 7,392 | 5,272 | 3,910 | 4,503 | 3,311 |
| | Nanoemulsion + Sulphoraphane | Average | 100,000 | 37,326 | 61,717 | 72,648 | 71,920 | 73,116 | 79,156 | 81,044 |
| | | Desvest | 5,917 | 3,409 | 2,817 | 2,084 | 2,230 | 3,624 | 3,844 | 3,125 |

Table 3

| | | | | PI | AnnexinV+PI | C neg | AnnexinV |
|---|---|---|---|---|---|---|---|
| 3T3 | | C - | | 2.70±0.14 | 1.40±0.28 | 91.50±0.14 | 4.40±0.57 |
| | Sulforaphane (mM) | | 12.5 | 4.30±0.54 | 3.07±0.67 | 81.57±3.40 | 11.07±1.59 |
| | | | 6.25 | 3.17±0.76 | 2.03±0.46 | 86.97±1.68 | 7.83±1.50 |
| | | | 3.125 | 4.10±2.38 | 1.93±0.15 | 86.23±1.77 | 7.73±1.23 |
| | Nanoemulsion + sulforaphane (mM, sulforaphane) | | 12.5 | 4.50±1.70 | 6.37±1.92 | 69.45±4.45 | 19.68±2.53 |
| | | | 6.26 | 2.13±0.50 | 237±0.61 | 82.53±3.81 | 12.97±4.06 |
| | | | 3.125 | 6.80±3.12 | 2.43±0.31 | 85.10±2.91 | 5.67±0.76 |
| | | | | | | | |
| HaCaT | | C - | | 4.40±4.38 | 3.45±3.18 | 89.40±0.14 | 5.75±0.64 |
| | Sulforaphane (mM) | | 12.5 | 2.70±0.30 | 2.03±0.35 | 88.10±3.40 | 7.17±1.74 |
| | | | 6.25 | 2.33±0.61 | 1.83±0.15 | 88.50±1.65 | 7.33±1.82 |
| | | | 3.125 | 1.87±0.81 | 1.27±0.06 | 90.97±1.77 | 5.90±0.98 |
| | Nanoemulsion + sulforaphane (mM, sulforaphane) | | 12.5 | 5.23±1.02 | 10.70±1.85 | 78.47±4.45 | 5.60±1.81 |
| | | | 6.26 | 2.43±0.32 | 12.01±2.15 | 72.53±3.81 | 12.97±5.74 |
| | | | 3.125 | 5.43±2.44 | 6.40±3.03 | 79.97±2.91 | 8.20±0.53 |

## Citation List

Hassan, P. et al, "Making sense of Brownian motion: colloid characterization by dynamic light scattering", Langmuir, 2015, vol. 31, pp.3-12.

Thirumdas, R. et al. "Health benefits of dietary glucosinolates and its derived isothiocyanates", Indian Food Industry, 2017, 36(2), 31-36.

## Claims

1. A liquid oil/in/water (O/W) nanoemulsion comprising a continuous aqueous phase and a fatty phase of dispersed oil droplets, said droplets having an average size comprised from 30 to 100 nm, and a zeta potential absolute value equal or higher than 30 mV.

2. The liquid O/W nanoemulsion of claim 1, which has a polydispersity index (PDI) comprised from 0.15 to 0.25, particularly from 0.17 to 0.22, particularly from 0.18 to 0.21.

3. The nanoemulsion of any one of the preceding claims, wherein:

   - the fatty phase is at % by weight with respect the total weight of the nanoemulsion comprised from 9 to 11% (w/w);
   - the aqueous phase is at a % by weight with respect the total weight of the nanoemulsion comprised from 75 to 85% (w/w); and

   the nanoemulsion further comprising a surfactant system:

   (a) which has a total hydrophilic-lipophilic balance (HLB) value from 11-15, and
   (b) is at a % by weight with respect to the total weight of the nanoemulsion comprised from 8 to 10% (w/w),

   provided that the sum of the components does not exceed the 100%.

4. The nanoemulsion of any one of the preceding claims, wherein the oil droplets encapsulate one or more poorly water-soluble compounds, wherein a compound is considered to be "poorly water-soluble" when an amount equal to or lower than 1 g, particularly equal to or lower than 0.1 g, is dissolved in 100 mL of water at 20°C.

5. The nanoemulsion of claim 4, wherein the poorly water-soluble compound is at a % by weight with respect to the total weight of the nanoemulsion comprised from 0.005 to 1 %, or from 0.0015 to 0.05%, or from 0.01 to 0.03%.

6. The nanoemulsion of any one of the preceding claims, wherein the surfactant system consists of two non-ionic surfactants selected from the group consisting of phospholipids, polyoxyethylene sorbitan fatty acid derivatives, castor oil or hydrogenated castor oil ethoxylates, fatty acid ethoxylates, alcohol ethoxylates, polyoxyethylene-polyoxypropylene copolymers, block co-polymers, alkylphenol surfactants and polyglycolized medium chain triglycerides, sorbitan esters, and combinations thereof; particularly selected from polyoxyethylene sorbitan esters, such as polyoxyethylene sorbitan monooleate, and sorbitan esters of fatty acids, such as sorbitan monooleate.

7. The nanoemulsion of any one of the preceding claims, wherein the fatty phase comprises long chain fatty acids having from 14 to 22 carbon atoms.

8. The nanoemulsion of claim 7, wherein the long chain fatty acids comprises one or more oxyl units of formula -[(C$_1$-C$_{20}$)alkylene-O]-, such as -[CH$_2$-CH$_2$-O]- units.

9. The nanoemulsion of any one of the preceding claims, which further comprises one or more of a steric stabilizer(s) and an antioxidant(s), wherein, the steric stabilizer is at a % by weight with respect to the total weight of the nanoemulsion comprised from 0.01 to 0.5% (w/w), and the antioxidant is at a % by weight with respect to the total weight of the nanoemulsion comprised from 0.5 to 2% (w/w).

10. The nanoemulsion of any one of the preceding claims, which is selected from the following nanoemulsions (a) to (e):

(a) - oleoyl polyoxyl-6 glyceride (such as Labrafil M 1944®): 9.5-10.5 % (w/w),

- water: 70-85% (w/w),
- surfactant system having a HLB value from 12.5 to 13.0: 8.5 to 9.5% (w/w), and
- optionally the one or more poorly water-soluble compound(s) at a % by weight comprised from 0.005 to 0.1% or from 0.01 to 0.05% or from 0.015 to 0.025%;

(b) - oleoyl polyoxyl-6 glyceride (such as Labrafil M 1944®): 9.5-10.5 %(w/w),

- water: 70-85% (w/w),
- the surfactant system has a HLB value from 12.5 to 13.0, and comprises a polyoxyethylene sorbitan fatty acid derivative and a sorbitan ester of a fatty acid: 8.5 to 9.5% (w/w), and
- optionally the one or more poorly water-soluble compound(s) at a % by weight comprised from 0.005 to 0.1% or from 0.01 to 0.05% or from 0.015 to 0.025%;

(c) - oleoyl polyoxyl-6 glyceride (such as Labrafil M 1944®): 9.5-10.5 %(w/w),

- water: 70-85% (w/w),
- the surfactant system has a HLB value from 12.5 to 13.00, and comprises polyoxyethylene sorbitan monooleate and sorbitan monooleate: 8.5 to 9.5% (w/w), and
- optionally the one or more poorly water-soluble compound(s) at a % by weight comprised from 0.005 to 0.1% or from 0.01 to 0.05% or from 0.015 to 0.025%;

(d) - oleoyl polyoxyl-6 glyceride (such as Labrafil M 1944 ®): 9.5-10.5% (w/w),

- water: 75-85% (w/w),
- surfactant system with a HLB value from 12.5 to 13: 8-10 % (w/w),
- steric stabilizer(s): 0.05-0.2 % (w/w),
- antioxidant(s): 0.5-1.5% (w/w), and
- optionally the one or more poorly water-soluble compound(s) at a % by weight comprised from 0.005 to 0.1% or from 0.01 to 0.05% or from 0.015 to 0.025%; and

(e) - oleoyl polyoxyl-6 glyceride (such as Labrafil M 1944 ®): 9.5-10.5% (w/w),

 - water: 75-85% (w/w),
 - surfactant system consisting of polyoxyethylene sorbitan monooleate and sorbitan monooleate, the system a HLB value from 12.8 to 12.9: 8-10 % (w/w);
 - poloxamer: 0.05-0.2 % (w/w),
 - vitamin(s), such as α-tocopherol: 0.5-1.5%, and
 - optionally the one or more poorly water-soluble compound(s) at a % by weight comprised from 0.005 to 0.1% or from 0.01 to 0.05% or from 0.015 to 0.025%;

provided that the sum of the components does not exceed the 100%,

11. The nanoemulsion of any one of the preceding claims, wherein the poorly water-soluble compound is a cosmetic or pharmaceutical and the nanoemulsion further includes one or more pharmaceutically or cosmetically acceptable excipients.

12. The nanoemulsion of any one of the preceding claims, wherein the poorly water-soluble compound is an isothiocyanate-based compound such as an allyl isothiocyanate, phenethyl isothiocyanate, or benzyl isothiocyanate, particularly sulforaphane.

13. A process for preparing a nanoemulsion of any one of the claims 1-11, which comprises the steps of (a) mixing the aqueous and fatty phases and the surfactant system as defined above, at a speed of at least 5000 rpm at room temperature, the fatty phase optionally including the one or more poorly water-soluble compound(s), and (b) sonicating or subjecting to ultrasounds the resulting coarse emulsion from step (a).

14. A nanoemulsion as defined in claim 1, for use as a delivery system of a poorly water-soluble pharmaceutical.

15. A nanoemulsion as defined in any one of the claims 1 to 12 encapsulating one or more poorly water-soluble compounds which are pharmaceutical for use in therapy.

16. A nanoemulsion as defined in any one of the claims 1 to 12 encapsulating one or more poorly water-soluble compounds which are pharmaceutical, for use in the treatment or prevention of cancer.

17. Use of a nanoemulsion as defined in claim 1 as a delivery system of a poorly water-soluble compound, wherein a compound is considered to be "poorly water-soluble" when an amount equal to or lower than 1 g, particularly equal to or lower than 0.1 g, is dissolved in 100 mL of water at 20°C.

Europäisches
Patentamt

European
Patent Office

Office européen
des brevets

# EUROPEAN SEARCH REPORT

Application Number

EP 20 38 2966

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/189387 A1 (SANOFI SA [FR]; UNIV SANTIAGO COMPOSTELA [ES]) 17 December 2015 (2015-12-17) * paragraphs [0001], [0007]; example 1; table 2 * * page 35, paragraph 1 * ----- | 1-8,11, 13-17 | INV. A61K9/107 A61K31/26 A61K47/14 A61K47/26 |

TECHNICAL FIELDS
SEARCHED (IPC)

A61K

The present search report has been drawn up for all claims

| Place of search | Date of completion of the search | Examiner |
|---|---|---|
| Munich | 27 April 2021 | Friederich, Pierre |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 20 38 2966

This annex lists the patent family members relating to the patent documents cited in the above-mentioned European search report.
The members are as contained in the European Patent Office EDP file on
The European Patent Office is in no way liable for these particulars which are merely given for the purpose of information.

27-04-2021

| Patent document cited in search report | Publication date | Patent family member(s) | Publication date |
|---|---|---|---|
| WO 2015189387 A1 | 17-12-2015 | EP 3154531 A1<br>JP 2017517549 A<br>US 2017087096 A1<br>WO 2015189387 A1 | 19-04-2017<br>29-06-2017<br>30-03-2017<br>17-12-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HASSAN, P. et al.** Making sense of Brownian motion: colloid characterization by dynamic light scattering. *Langmuir,* 2015, vol. 31, 3-12

- **THIRUMDAS, R. et al.** Health benefits of dietary glucosinolates and its derived isothiocyanates. *Indian Food Industry,* 2017, vol. 36 (2), 31-36